(19) **Europäisches Patentamt**
**European Patent Office**
**Office européen des brevets**

(11) **EP 1 759 209 B1**

(12) **EUROPÄISCHE PATENTSCHRIFT**

(45) Veröffentlichungstag und Bekanntmachung des Hinweises auf die Patenterteilung:
**20.08.2008 Patentblatt 2008/34**

(21) Anmeldenummer: **05742260.2**

(22) Anmeldetag: **10.05.2005**

(51) Int Cl.:
*G01N 33/538* *(2006.01)*    *G01N 33/543* *(2006.01)*

(86) Internationale Anmeldenummer:
**PCT/EP2005/005016**

(87) Internationale Veröffentlichungsnummer:
**WO 2005/111607 (24.11.2005 Gazette 2005/47)**

(54) **VERFAHREN ZUR ERHÖHUNG DES DYNAMISCHEN MESSBEREICHS VON AUF SPEZIFISCHEN BINDEREAKTIONEN BASIERENDEN, INSBESONDERE IMMUNOLOGISCHEN TESTELEMENTEN**

METHOD FOR INCREASING THE DYNAMIC MEASURING RANGE OF TEST ELEMENTS, ESPECIALLY IMMUNOLOGICAL TEST ELEMENTS, THAT ARE BASED ON SPECIFIC BONDING REACTIONS

PROCEDE POUR ELARGIR LA PLAGE DE MESURE DYNAMIQUE D'ELEMENTS SERVANT A REALISER DES TESTS NOTAMMENT IMMUNOLOGIQUES, BASES SUR DES REACTIONS DE FIXATION SPECIFIQUES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **12.05.2004 DE 102004023402**

(43) Veröffentlichungstag der Anmeldung:
**07.03.2007 Patentblatt 2007/10**

(73) Patentinhaber:
• **Roche Diagnostics GmbH**
  **68305 Mannheim (DE)**
  Benannte Vertragsstaaten:
  **DE**
• **F. Hoffmann-La Roche AG**
  **4070 Basel (CH)**
  Benannte Vertragsstaaten:
  **AT BE BG CH CY CZ DK EE ES FI FR GB GR HU IE IS IT LI LT LU MC**

(72) Erfinder:
• **SPINKE, Juergen**
  **64653 Lorsch (DE)**
• **SCHAEFFLER, Juergen**
  **69469 Weinheim (DE)**
• **BODENBACH, Ludger**
  **69253 Heiligkreuzsteinach (DE)**

(56) Entgegenhaltungen:
**EP-A- 1 361 435**        **US-A1- 2002 055 126**
**US-A1- 2003 119 204**

**Beschreibung**

Die Erfindung betrifft ein Verfahren zur Erhöhung des dynamischen Messbereichs von auf spezifischen Bindereaktionen basierenden, insbesondere immunologischen Testelementen, insbesondere von optisch auswertbaren immunologischen Chromatographieteststreifen.

Immunologische Teststreifen stellen ein weit verbreitetes Hilfsmittel zur schnellen Bestimmung von Drogen, Schwangerschaftshormonen, Infektionskrankheiten, oder so genannten "Cardiac Markern", wie Troponin T, dar. Dabei haben sowohl qualitative Tests, die rein visuell ausgelesen werden und oft nur eine "Ja-Nein"-Antwort liefern, als auch quantitative Tests, die mittels eines Auslesegerätes ausgewertet werden, breite Anwendung gefunden.

Schnelltests für immunologisch nachweisbare Substanzen sind für eine Vielzahl von unterschiedlichen Parametern seit langem bekannt, beispielsweise aus WO 97/06439, EP 0 291 194, US 5,591,645, US 4,861,711, US 5,141,850, US 6,506,612, US 5,458,852, US 5,073,484. Hier werden meist die immunologischen Nachweisreagenzien (im Wesentlichen markierte und unmarkierte Antikörper bzw. Antigene) in trockener Form auf einem Träger bereitgestellt, der den Transport einer Probenflüssigkeit (insbesondere Körperflüssigkeiten wie Blut, Serum, Plasma, Urin, Speichel etc.) auf oder in dem Träger erlaubt. Vorzugsweise ist der Träger hierzu kapillaraktiv, beispielsweise eine Membran oder ein mit Kapillarkanälen versehener Kunststoffträger (wie z. B. in US 5,458,852). In der Fachwelt spricht man oft von immunologischen oder immunchromatographischen Teststreifen oder Testdevices. Diese Begriffe sowie der Ausdruck "trägergebundene immunologische Tests" oder "trägergebundene immunologische Testelemente" werden oftmals synonym verwendet und sollen auch im Folgenden austauschbar sein.

Die Auswertung solcher immunologischer Testdevices erfolgt bei einfachen Systemen und insbesondere bei rein qualitativen Analysen (wo nur die Aussage "der Analyt ist vorhanden bzw. nicht vorhanden" von Interesse ist) oftmals rein visuell. Insbesondere im Bereich der Schwangerschaftschnelltests hat sich dieses Prinzip im Markt breit durchgesetzt.

(Semi-)Quantitative immunologische Schnelltests werden meist mit Hilfe entsprechender, auf die jeweiligen Teststreifen abgestimmter Messgeräte ausgewertet. Je nach Art der zur Detektion des Analyten verwendeten Markierung der Reagenzien des Testdevices werden unterschiedliche Messprinzipien verwendet. Weit verbreitet und einfach zu handhaben sind optische Detektionsmethoden, insbesondere die Messung von Remission und Fluoreszenz.

Viele Systeme aus dem Stand der Technik sorgen dafür, dass Analytnachweiszone (im Nachfolgenden auch kurz: "Nachweiszone") und Kontrollzone räumlich eng begrenzt und deutlich voneinander getrennt auf dem Testdevice angeordnet sind. Zweckmäßig hat sich hierzu vor allem erwiesen, entsprechende Bindereagenzien in Linien- oder Strichform auf dem Testdevice anzubringen. Im Messgerät zur Auswertung der Testdevices befinden sich zum Zweck der Auswertung der Analytnachweis- und Kontrollzonen deshalb oftmals ortsauflösende optische Systeme wie z. B. Kamerachips, oder 2- oder 3-dimensionale Photodiodenarrays. Die Signale der optischen Systeme werden dann von einer entsprechenden Auswertesoftware in Konzentrationswerte umgewandelt und zur Anzeige gebracht.

Bei den immunologischen Testdevices des Standes der Technik ist es nicht möglich, beliebige Konzentrationen des Analyten in der Probe quantitativ zu erfassen. Nach unten hin, d. h. im Hinblick auf die untere Nachweisgrenze, ist der Messbereich beispielsweise durch die Affinität und Selektivität der verwendeten Bindepartner (meist Antikörper) und die Empfindlichkeit der Nachweisoptik im Hinblick auf die verwendeten Markierungen (Label) beschränkt. Nach oben hin, d. h. im Hinblick auf den dynamischen Messbereich, limitieren Sättigungseffekte den Messbereich. So ist es bei Analyten, die in sehr hohen Konzentrationen in der Probe vorkommen können, oftmals nicht möglich, eine adäquate Menge an Bindepartner im Testdevice zur Verfügung zu stellen. Insbesondere in den Analytnachweis- und Kontrollzonen, wo Bindepartner räumlich eng begrenzt auf dem Testdevice angeordnet sind, kann nicht beliebig viel Bindepartner untergebracht werden. Dies kann insbesondere in den Fällen problematisch sein, in denen sowohl eine niedrige Nachweisgrenze für den Analyten erforderlich ist (und man deshalb bestrebt ist, die Bindepartner in der Nachweiszone möglichst stark konzentriert, d. h. auf engem Raum, unterzubringen und so aufgrund der nur begrenzten Verfügbarkeit von Bindeplätzen auf dem Testdevice nur eine verhältnismäßig geringe Menge an Bindepartner bereitstellen kann), der Analyt aber in stark schwankenden Mengen in der Probe vorliegen kann, also sowohl sehr niedrige als auch sehr hohe Analytkonzentrationen vorkommen können. Bei hohen Analytkonzentrationen erreicht man eine Absättigung der Nachweiszone mit entsprechenden Nachweisreagenzien, so dass ein Sättigungsverhalten der Analytkonzentration-Detektionssignal-Beziehung resultiert: oberhalb einer bestimmten Analytkonzentration kommt es zu keinem Anstieg des Detektionssignals; die Auswertekurve flacht ab und kann nicht mehr sinnvoll ausgewertet werden.

Erschwerend kommt hinzu, dass vor allem bei Sandwich-Immunoassays bei sehr hohen Analytkonzentrationen nicht nur ein Abflachen der Kurve zu beobachten ist, welche die Beziehung zwischen Analytkonzentration und Detektionssignal wiedergibt, sondern sogar eine Abnahme des Signals mit steigenden Analytkonzentrationen. Man spricht hier vom so genannten "High-Dose-Hook-Effect": Bei sehr hohen Analytkonzentrationen wird beobachtet, dass die Signalintensität bei Sandwich-Immunoassays, die zunächst mit steigender Analytkonzentration zunimmt, wieder abnimmt. Man erklärt sich das dadurch, dass die im Test angebotene Antikörpermenge nicht mehr ausreicht, um in jedem Fall mit den Analytmolekülen (Antigenen) einen Sandwich-Komplex (d. h. einen Komplex mit zwei Antikörpern je Antigen) zu bilden. Es bilden sich vermehrt Komplexe aus Analyt und jeweils einem Antikörper, die jedoch für sich genommen nicht mehr detektiert werden. So kann es unter Umständen zu falsch negativen oder zu niedrigen Messergebnissen kommen, was natürlich zu vermeiden ist.

Speziell die quantitativen immunologischen Teststreifen, bei denen ein Signal über Remissionsmessungen bestimmt wird, zeigen gegenüber herkömmlichen, meist in Großlabors eingesetzten Analysensystemen zum Teil noch deutliche Schwächen. Besonders die Präzision und der dynamische Messbereich sind bei den Teststreifen meist schlechter. Dies limitiert speziell das Anwendungsgebiet der hoch sensitiven Sandwichassays, beispielsweise für das Therapiemonitoring, wo ein möglichst großer Messbereich erwünscht ist.

Hinzu kommt, dass bei manchen Parametern, wie z. B. Myoglobin oder D-Dimer, einerseits eine niedrige Nachweisgrenze erforderlich ist, andererseits sehr hohe Konzentrationen dieser Analyten im Probenmaterial, teilweise weit oberhalb der Entscheidungsgrenze "normal-pathologisch", vorkommen können. In diesen Fällen wäre es wünschenswert, Testdevices zur Verfügung zu haben, die einen möglichst großen Messbereich aufweisen, um ohne Probenvorverdünnung verlässliche Messwerte zu erhalten. Insbesondere wäre dies von Vorteil für den Einsatz solcher Testdevices bei der Verlaufskontrolle von entsprechenden Krankheitsbildern.

Im Stand der Technik mangelt es nicht an Konzepten, die oben beschriebenen Probleme zu lösen. Allerdings vermag bislang keiner der Vorschläge in allen Punkten zu überzeugen. Insbesondere ist die Umsetzung der Konzepte im Bereich der immunochromatographischen Testdevices bislang nicht befriedigend gelungen.

US 6,248,597 beschreibt einen auf Lichtstreuung basierenden heterogenen Agglutinations-Immunoassay, bei dem der dynamische Messbereich dadurch erweitert wird, dass eine Mischung aus Partikeln mit unterschiedlichen Streueigenschaften verwendet wird. Dabei sind auf den Partikeln, die eine große Lichtstreuung bewirken, Bindepartner mit hoher Affinität für den Analyten immobilisiert. Auf den Partikeln, die eine geringe Lichtstreuung bewirken, sind hingegen Bindepartner mit niedriger Affinität für den Analyten immobilisiert.

Ein ähnliches Verfahren ist aus US 5,585,241 bekannt. Hier wird im Zusammenhang mit einem Durchflusszytometrie-Immunoassay zur Steigerung des dynamischen Messbereichs vorgeschlagen, zwei unterschiedlich große Partikel mit zwei unterschiedlich affinen Antikörpern gegen dasselbe Antigen zu beladen (kleine Partikel mit hochaffinem Antikörper; große Partikel mit niedrigaffinem Antikörper) und zur Detektion des Antigens über die Bildung eines Sandwich-Komplexes einen weiteren, detektierbar markierten Antikörper zu verwenden. Das vorgeschlagene System arbeitet dabei mit zwei unterschiedlichen Standardkurven (eine für jede Partikelsorte) und erlaubt die quantitative Analytbestimmung über eine ausgeklügelte Software.

Zur Vermeidung des Hook-Effekts bei hohen Analytkonzentrationen ("high dose hook effect") offenbart US 4,743,542 ein Verfahren, bei dem neben einem detektierbar markierten Antikörper gegen das Zielantigen einfach eine bestimmte Menge des selben, allerdings unmarkierten Antikörpers zur Probe gegeben wird. Dadurch konkurrieren beide Antikörper um die Analytmoleküle und die für den Hook-Effekt typische Übersättigung tritt - wenn überhaupt - erst bei höheren Antigenkonzentrationen auf. Dadurch wird der dynamische Messbereich zu höheren Konzentrationen hin erweitert, allerdings auf Kosten der Sensitivität. Gleichwirkend hierzu wird die Verwendung niedrigaffinerer Antikörper vorgeschlagen.

US 4,595,661 beschreibt heterogene Sandwich-Immunoassays, bei denen der Hook-Effekt dadurch vermieden wird, dass neben einem immobilisierten Fängerantikörper zwei lösliche Antikörper verwendet werden, die unterschiedliche Affinität und Spezifität für das Antigen aufweisen. Der Antikörper mit geringerer Affinität trägt dabei nur bei hohen Antigenkonzentrationen merklich zum Messsignal bei und vermeidet so, dass der Hook-Effekt bemerkbar wird.

Aus US 5,073,484 ist es bekannt, mit Hilfe von mehreren diskreten, in einem durchströmungsfähigen Träger nacheinander liegenden Bindezonen einen immunologisch nachweisbaren Analyten quantitativ nachzuweisen. Je mehr Analyt in der Probe vorhanden ist, in desto mehr Zonen finden die spezifischen Binde- und Nachweisreaktionen statt. Die Anzahl der Zonen, die nach Probenkontakt eine Färbung aufweisen, korreliert dabei mit der Menge an Analyt in der Probe. US 5,073,484 schlägt zur Erhöhung der Genauigkeit und zur Erweiterung des Messbereichs vor, die Anzahl der Bindezonen zu erhöhen. Nachteilig hieran ist, dass eine automatische Auswertung der Bindezonen eine relativ aufwändige Optik erfordert, die in der Lage ist, unter Umständen eine Vielzahl von Zonen gleichzeitig zu erfassen und auszuwerten, um so eine quantitative Analytbestimmung zu erlauben. Zudem müssen die Testdevices aufgrund der relativ großen Anzahl an diskreten, voneinander räumlich getrennten Bindezonen verhältnismäßig lang sein. Um ein sicheres Durchwandern der Probe durch das Testdevice zu gewährleisten muss deshalb mit relativ großen Probenvolumina gearbeitet werden, was insbesondere für den Fall, dass Vollblutproben verwendet werden sollen, insbesondere aus Gründen der Probengewinnung ebenfalls nachteilig ist.

WO 00/31538 beschreibt immunochromatographische Teststreifen, bei denen neben einer Analytnachweiszone eine oder mehrere Kontrollzonen auf einer saugfähigen Matrix untergebracht sind. Mit einer detektierbaren Markierung versehene Bindepartner für den Analyten werden sowohl in der Analytnachweiszone, als auch in den Kontrollzonen an die Matrix gebunden. In den Kontrollzonen werden dabei genau definierte Mengen an markiertem Bindepartner gebunden, wobei diese Mengen unabhängig von der Menge an Analyt in der Probe sind. Vorzugsweise werden in den Kontrollzonen unterschiedliche Mengen an markiertem Bindepartner gebunden, so dass auf dem Teststreifen quasi interne Vergleichsskalen enthalten sind. Bei der Auswertung der Analytnachweiszone werden die Kontrollzonen zur Kalibration herangezogen. Zur Erhöhung des dynamischen Messbereichs, insbesondere für nichtlineare Konzentrations-Messsignal-Beziehungen, schlägt WO 00/31538 vor, zusätzliche Kontrollzonen auf dem Teststreifen vorzusehen.

US 2003/0119204 beschreibt immunochromatographische Teststreifen, welche neben einer Analytnachweiszone zwei oder mehr Kontrollzonen auf einer saugfähigen Matrix enthalten. Die zwei oder mehr Kontrollzonen dienen hierbei zur Erstellung einer internen Kalibration, indem deren Intensitätswerte auf dem Teststreifen bestimmt und mit vorbekannten Analytkonzentrationen in Beziehung gesetzt werden, so dass daraus eine Kalibrationskurve beziehungsweise eine Kalibrationsbeziehung erstellt wird. Mit deren Hilfe wird in einem nachfolgenden Schritt ausgehend von der bestimmten Intensität der Analytnachweiszone die Analytkonzentration ermittelt. Eine Erhöhung des dynamischen Messbereichs zu höheren Analytkonzentrationen, bei denen Sättigungseffekte den Messbereich limitieren, ist mit diesen Verfahren nicht möglich.

Aus J. Hampl et al., "Upconverting Phosphor Reporters in Immunochromatographic Assays", Analytical Biochemistry 288, 176 - 187 (2001) ist es bekannt, in immunochromatographischen Teststreifen, die Fluoreszenzlabel zur Analytdetektion nutzen, neben der eigentlichen Nachweiszone ("target line"), die einen analytspezifischen Antikörper in immobilisierter Form enthält, auch die Kontrollzone ("control line"), die einen speziesspezifischen Antikörper in immobilisierter Form enthält, zur Auswertung des Messsignals heranzuziehen. Eine analoge Anwendung ist von OraSure Technologies, Inc., Bethlehem, PA, U.S.A., auf www.orasure.com beschrieben. Die Auswertung sowohl der "target line" als auch der "control line" geschieht primär deshalb, um Variationen im Messsignal, die durch die tatsächliche Menge an Flüssigkeit im optisch vermessenen Bereich des Teststreifens bedingt sind, zu eliminieren. Indirekt wird dadurch auch die Sensitivität des Nachweisverfahrens (Assays) erhöht (es erfolgt also eine Erweiterung des dynamischen Messbereichs zu niedrigeren Konzentrationen hin). Eine Erweiterung des dynamischen Messbereichs zu höheren Konzentrationen hin wird hingegen nicht berichtet.

Eine Erweiterung des dynamischen Messbereichs von immunochromatographischen Testdevices ist de facto auch dadurch zu erreichen, dass man das Probenmaterial entsprechend vor der Analyse verdünnt. Eine solchermaßen erzielte Messbereichserweiterung ist jedoch nur unbefriedigend, da hierzu ein zusätzlicher, potentiell zu Fehlern in der Analyse führender Handhabungsschritt benötigt wird. Außerdem ist insbesondere in den Fällen, in denen ein Analyt in ähnlichen Proben möglicherweise sowohl in sehr hohen als auch in sehr niedrigen Konzentrationen vorkommen kann, eine kontrollierte Probenverdünnung nur dann ratsam, wenn der Analyt in hohen Konzentrationen in der Probe vorliegt, nicht jedoch im umgekehrten Fall, da hier sonst unter Umständen die untere Nachweisgrenze durch die Verdünnung unterschritten wird und der Analyt in der Probe fälschlich nicht erfasst wird.

Für immunochromatographische Testdevices mangelt es bislang an einfachen und zuverlässigen Möglichkeiten, den dynamischen Messbereich hin zu höheren Analytkonzentrationen zu erweitern, ohne die untere Nachweisgrenze für den Analytnachweis zu beeinträchtigen.

Der vorliegenden Erfindung liegt die Aufgabe zugrunde, die Nachteile des Standes der Technik zu beseitigen. Insbesondere ist es Aufgabe der vorliegenden Erfindung, den dynamischen Messbereich von auf spezifischen Bindereaktionen basierenden, insbesondere immunologischen Testelementen hin zu höheren Analytkonzentrationen zu erweitern, wobei dies insbesondere dergestalt ermöglicht werden sollte, dass keine Einbußen im Hinblick auf die untere Nachweisgrenze hingenommen werden müssen.

Diese Aufgabe wird durch den Gegenstand der Erfindung gelöst.

Gegenstand der Erfindung ist ein Verfahren gemäß Anspruch 1 und eine Verwendung gemäß Anspruch 9. Bevorzugte Ausführungsformen der Erfindung sind Gegenstand der abhängigen Ansprüche.

Die Erfindung ermöglicht es, den dynamischen Messbereich von auf spezifischen Bindereaktionen basierenden, insbesondere immunologischen Testelementen hin zu höheren Analytkonzentrationen zu verschieben, ohne Einbußen im Hinblick auf die Nachweisgrenze hinnehmen zu müssen. Dazu wird erfindungsgemäß vorgeschlagen, zumindest zwei Zonen in oder auf dem Testelement bereitzustellen, die Reagenzien enthalten, die aufgrund unterschiedlicher Affinität zum Analyten (beispielsweise im Fall unterschiedlich affiner Antikörper gegen den Analyten) oder aufgrund unterschiedlicher Prinzipien der Wechselwirkung mit dem Analyten oder mit anderen am Analytnachweis beteiligten Reagenzien (beispielsweise Antikörper gerichtet gegen den Analyten in einer Zone und Analyt oder Analytanalogon in einer anderen Zone) unterschiedlich starke detektierbare Signale erzeugen. "Unterschiedliche Prinzipien der Wechselwirkung" können dabei z. B. unterschiedliche Testprinzipien sein, beispielsweise Sandwichkomplexbildung in einer Zone und kompetitive Testführung in der anderen Zone. Zur Auswertung der Analytkonzentration-Signalstärke-Beziehung werden die Signale in den zumindest zwei Zonen herangezogen und durch ein geeignetes Verfahren (Korrelation) zur Analytbestimmung herangezogen.

Die zwei im erfindungsgemäßen Verfahren bedeutenden Zonen in oder auf dem Testelement sollen der besseren Verständlichkeit und Unterscheidbarkeit wegen nachfolgend als Analytnachweiszone (kurz: Nachweiszone) und Kontrollzone bezeichnet werden. Diese Bezeichnung soll auch beibehalten werden, wenn sie nach herkömmlichem Sprachgebrauch unüblich ist, beispielsweise wenn in den Zonen unterschiedlich affine Bindepartner untergebracht sind und sich ein Signal deshalb erst ab einer Schwellenkonzentration an Analyt beobachten lässt.

Die Erfindung umfasst auch Verfahren, bei denen mehr als 1 Nachweiszone und/oder mehr als 1 Kontrollzone auf einem Testelement ausgewertet werden. Beispielsweise kann das Verfahren auch eingesetzt werden zur Auswertung von Testelementen, die einen Nachweisstrich mit hochaffinen Bindepartnern, einen Nachweisstrich mit niedrigaffinen Bindepartnern und eine Kontrollzone (enthaltend z. B. immobilisiertes Analytanalogon) enthalten.

Die erfindungsgemäße Lösung betrifft insbesondere die Erweiterung des Messbereiches von immunologischen Testdevices durch die zusätzliche quantitative Auswertung des Kontrollstriches bei Sandwichassays. Dieser dient üblicherweise nur als Funktionskontrolle für den Anwender und wird nicht zur Quantifizierung des Analyten herangezogen. Mit zunehmendem Analytgehalt wird jedoch immer mehr Antikörper-Label-Konjugat am Signalstrich abgefangen oder mit Analyt abgesättigt, so dass immer weniger Antikörper-Label-Konjugat (z.B. Antikörper-Gold-Konjugat) am Kontrollstrich gebunden wird. Der Kontrollstrich nimmt daher mit zunehmender Analytkonzentration an Signalstärke ab. Durch gleichzeitige Messungen der Signalstärke (beispielsweise durch Remissions- oder Fluoreszenzmessung) am Kontrollstrich und am Signalstrich und der Verrechnung der beiden Signalintensitäten mit einem geeigneten Algorithmus lassen sich der dynamische Messbereich sowie die Steigung der Kalibrationskurve (und damit auch der Präzision bei höheren Konzentrationen) deutlich verbessern.

Analoges gilt natürlich auch für Testelemente, die auf anderen als immunologischen spezifischen Bindereaktionen basieren. Entsprechend spezifische Bindereaktionen sind dem Fachmann bekannt. Beispielhaft genannt seien die Bindepaare:

Antikörper mit Hapten, Antigen oder anderen Antikörpern (beispielsweise speziesspezifische Antikörper-Antikörper-Wechselwirkungen), wobei teilweise auch jeweils
Fragmente dieser Spezies genügen;
Biotin mit Avidin oder Streptavidin;
Hormon mit Hormonrezeptor;
Zucker mit Lektin;
Nukleinsäure mit komplementärer Nukleinsäure; und dergleichen mehr.

Der besseren Verständlichkeit und Übersichtlichkeit wegen soll im Folgenden verstärkt auf immunologische Bindepaare, d. h. auf die Bindepaare Antikörper mit Hapten bzw. Antigen bzw. Antikörper eingegangen werden, ohne dass dies jedoch beschränkend auf diese zwar bevorzugte, aber dennoch nicht einzige Ausführungsform der Erfindung sein soll.

Das erfindungsgemäße Verfahren dient insbesondere zur Erweiterung des dynamischen Messbereiches zu höheren Analytkonzentrationen, bei denen Sättigungseffekte den Messbereich limitieren, insbesondere der "High-Dose Hook-Effect" auftritt, ohne Beeinflussung der unteren Nachweisgrenze mit Hilfe eines immunologischen Testelementes. Das Testelement weist dabei sowohl eine Analytnachweiszone als auch eine Kontrollzone auf. Die Probe wird mit dem Testelement und mit für den Analyten spezifischen Reagenzien in Kontakt gebracht und der Analyt führt - sofern er in der Probe vorhanden ist - durch Wechselwirkung mit den spezifischen Reagenzien zu einem in der Analytnachweiszone detektierbaren Signal. Das Messsignal ist von der Menge an Analyt in der Probe abhängig. Ein Teil der spezifischen Reagenzien, der nicht mit dem Analyten bzw. den Reagenzien in der Nachweiszone in Wechselwirkung getreten ist, führt in der Kontrollzone zu einem detektierbaren Signal. Wichtig ist dabei, dass dieses in der Kontrollzone detektierbare Signal ebenfalls von der Menge an Analyt in der Probe abhängig ist. Die Signale in der Analytnachweiszone und in der Kontrollzone werden gemessen und durch einen geeigneten Algorithmus, beispielsweise miteinander, verrechnet. Das Ergebnis der Verrechnung wird mit einer Eichkurve verglichen, und schließlich wird die Analytkonzentration bestimmt.

Erfindungsgemäß geeignete Analyten sind Analyten, die sich aufgrund einer spezifischen Bindepaarbeziehung nachweisen lassen. Insbesondere für den bevorzugten Fall des immunologischen Nachweises sind dies Antikörper, Antigene, Haptene (jeweils inkl. Fragmenten hiervon). Besonders bevorzugt sind die immunologisch nachweisbaren Analyten hCG, BNP, (NT-)proBNP, Troponin I, Troponin T, Myoglobin, D-Dimer, CRP, HIV, HCV, CD40, CK-MB, TSH, etc.

Als Probe, aus der die Analyten bestimmt werden können, sind erfindungsgemäß alle flüssigen oder in flüssige Form überführbaren Probenmaterialien geeignet. Insbesondere sind Körperflüssigkeiten wie Blut und daraus abgeleitete Fraktionen (Serum, Plasma), Speichel, Urin, zerebrospinale Flüssigkeit, Sperma, interstitielle Flüssigkeit, Schweiß und dergleichen mehr geeignet. Geeignet sind auch an sich nicht flüssige, jedoch durch Lösen oder Suspendieren in Lösungsmitteln, insbesondere wässrigen Lösemitteln, in die flüssige Phase überführbaren Probenmaterialien.

Die erfindungsgemäß einsetzbaren immunologischen Testelemente sind dem Fachmann an sich bekannt. Der Analytnachweis mit Hilfe solcher Testelemente beruht auf einer spezifischen Wechselwirkung zwischen dem Analyten und einem Bindepartner. Solche Wechselwirkungen umfassen die Bindepaare Antigen/Antikörper, Antikörper/Antikörper, Hapten/Antikörper, Antigenfragment/Antikörper, Antikörperfragment/Antikörper etc. Wie bereits eingangs erwähnt enthalten die Testelemente meist ein durchströmungsfähiges Material (z. B. Papier, Vlies, Membran, Kapillarkanal), das ggf. auf einem inerten Träger befestigt ist. Typischerweise weist ein Testelement je eine oder mehrere Probenaufgabezonen, Saugzonen, Chromatographiezonen, Nachweiszonen, Reaktionszonen, und Kontrollzonen auf. Wichtig für die Erfindung ist lediglich, dass zumindest eine (Analyt-)Nachweiszone und zumindest eine Kontrollzone vorhanden sind.

Als Analytnachweiszone dient typischerweise ein räumlich eng begrenzter, von der Kontrollzone separierter Bereich in oder auf dem durchströmungsfähigen Material, in dem im Verlauf der bestimmungsgemäßen Benutzung des Testelementes eine Spezies, die ein Maß für den Analyten ist, so gebunden wird, dass sie visuell, optisch oder auf andere Art und Weise detektiert werden kann. Typischerweise wird ein nachweisbarer Bindepartner für den Analyten, beispielsweise ein entsprechend markierter Anti-Analyt-Antikörper, über eine spezifische Wechselwirkung in der Analytnachweiszone gebunden. Zu diesem Zweck befindet sich in der Analytnachweiszone ein entsprechender, immobilisierter Bindepartner, beispielsweise ein Antikörper gegen den Analyten (so dass sich ein detektierbarer Sandwichkomplex aus immobilisiertem Antikörper, Analyt und detektierbar markiertem Antikörper bilden kann) oder eine Spezies eines anderen Bindepaares, beispielsweise immobilisiertes (Poly-)(Strept-)Avidin (so dass sich ein zuvor gebildeter Sandwichkomplex aus biotinyliertem Antikörper, Analyt und detektierbar markiertem Antikörper bilden kann). Aufbau, Funktion und weitere Abwandlungen solcher Nachweiszonen sind dem Fachmann an sich bekannt.

Als Kontrollzone dient typischerweise ein räumlich eng begrenzter, von der Analytnachweiszone separierter und meist stromabwärts von dieser gelegener Bereich auf oder in dem durchströmungsfähigen Material, in dem im Verlauf der bestimmungsgemäßen Benutzung des Testelementes unabhängig von der Anwesenheit des Analyten in der Probe eine Spezies so gebunden wird, dass sie visuell, optisch oder auf andere Art und Weise detektiert werden kann. Die Kontrollzone dient üblicherweise der Funktionskontrolle des Testelements. Ein Signal in der Kontrollzone belegt, dass Probe ordnungsgemäß den durchströmungsfähigen Träger durchströmt hat und idealerweise dass die entsprechenden Bindereagenzien funktionsfähig sind. Typischerweise wird ein nachweisbarer Bindepartner für den Analyten, beispielsweise ein entsprechend markierter Anti-Analyt-Antikörper, über eine spezifische Wechselwirkung in der Kontrollzone gebunden. Zu diesem Zweck befindet sich in der Kontrollzone ein entsprechender, immobilisierter Bindepartner, beispielsweise ein Antikörper gegen den markierten Anti-Analyt-Antikörper (so dass sich ein detektierbarer Komplex aus immobilisiertem Antikörper und detektierbar markiertem Antikörper bilden kann) oder ein immobilisiertes Analytanalogon (so dass sich ein Komplex aus Analyt und detektierbar markiertem Antikörper bilden kann). Aufbau, Funktion und weitere Abwandlungen solcher Kontrollzonen sind dem Fachmann an sich bekannt.

Die im Testelement enthaltenen oder zum Testelement bzw. zur Probe hinzuzufügenden spezifischen Reagenzien (synonym auch als "spezifische Bindepartner" bezeichnet) gehen eine selektive (Binde-)Reaktion mit dem Analyten oder den immobilisierten Bindepartnern auf dem Träger ein. Sie lassen direkt oder indirekt Rückschluss auf die in der Probe vorhandene Analytmenge zu.

Bevorzugte Bindepartner sind Antikörper (AK; im Englischen antibodies oder AB), insbesondere polyklonale Antikörper (PAK; im Englischen polyclonal antibodies oder PAB) oder monoklonale Antikörper (MAK; im Englischen monoclonal antibodies oder MAB), sowie Antigene und Haptene, sowie Fragmente hiervon, sofern sie für die Zwecke des spezifischen Analytnachweises aktiv sind.

Vorzugsweise wird ein Teil der Bindepartner so auf dem Testdevice zur Verfügung gestellt, dass sie durch die Probenflüssigkeit von diesem abgelöst werden können, beispielsweise durch Imprägnierung geeigneter Trägermaterialien wie Vliese, Membranen etc., oder durch Aufbringen und Trocknen in entsprechende (Kapillar-)Kanalstrukturen.

Es ist jedoch auch möglich, zumindest einen der Bindepartner in Form gelöster Reagenzien zum Schnelltest zuzugeben, beispielsweise die Probe mit der Reagenzienlösung zu versetzen oder die Reagenzienlösung unabhängig von der Probe auf das Testdevice aufzubringen. Erfindungsgemäß ist es auch möglich, wenn auch weniger bevorzugt, alle spezifischen Bindepartner in einer Lösung oder in mehreren Lösungen zum Schnelltest einzusetzen. Auf dem Testdevice befindet sich dann in der Nachweiszone lediglich ein weiterer Bindepartner, der den entsprechend markierten spezifischen Bindepartner abfangen kann und so eine indirekte Bindung des Analyten an eine Festphase des Schnelltests bewirkt. Analog befindet sich in der Kontrollzone ein Bindepartner, der den entsprechend markierten spezifischen Bindepartner abfangen kann, ohne dass eine direkte Beteiligung des Analyten erforderlich ist.

Sowohl in der Nachweiszone als auch in der Kontrollzone führt prinzipiell einer der dort immobilisierten Bindepartner zu einem detektierbaren Signal. Dabei ist es möglich, wenngleich nicht bevorzugt, dass die Signale in den beiden Zonen auf unterschiedlichen Prinzipien beruhen. Bevorzugt ist hingegen, dass sowohl das Signal in der Analytnachweiszone als auch in der Kontrollzone auf den selben Prinzipien beruht. Detektierbare Signale sind beispielsweise optisch oder visuell erfassbare Farbänderungen, Lumineszenz-, insbesondere Fluoreszenzsignale, radioaktive Strahlung und dergleichen mehr. Das detektierbare Signal wird dabei von einer entsprechend markierten Spezies (Bindepartner) erzeugt, welches - wie oben dargelegt - in der Analytnachweis- oder Kontrollzone gebunden wird. Als Markierung des Bindepartners kommen erfindungsgemäß unter anderem in Frage: partikuläre Markierungen, wie z. B. die Verwendung von farbigen Latices, Polymerlabel, oder Halbleiter-Nanokristallen (so genannten Quantum Dots) oder Metall(sol) label (Gold, Selen etc.), sowie nicht-partikuläre Markierungen (Enzym-, Radioisotop-, Fluoreszenzlabel) und dergleichen mehr.

Je nach verwendetem Label sind naturgemäß auch andere Detektionsmethoden erforderlich und möglich (z. B. Fluoreszenzmessung, Radioaktivitätsmessung, Enzymaktivitätsbestimmung etc.). Mit diesen Detektionsmethoden lassen sich die in der Analytnachweiszone und Kontrollzone erzeugten Signale messen, insbesondere mit entsprechend aufgebauten Messinstrumenten. Diese sind dem Fachmann aus dem Stand der Technik bekannt. Erfindungsgemäß wichtig ist aber, dass sowohl das Signal in der Analytnachweiszone als auch das in der Kontrollzone vom Messgerät erfasst werden. Geeignete Messgeräte und Verfahren zur Auswertung von Testelementen sind dem Fachmann aus dem Stand der Technik bekannt. Als typisches Beispiel für ein Messgerät sei das System "Cardiac Reader" von Roche Diagnostics GmbH, Mannheim, genannt. Hier wird ein immunologischer Teststreifen mittels einer oder mehrerer Lichtquellen (z. B. LEDs) beleuchtet und via ortsaufgelöster Remissionsmessung die Grauwerte der Nachweiszone (Signalstrich) und der Kontrollzone bestimmt. Ein entsprechendes Mess- und Auswerteverfahren ist beispielsweise aus US 5,717,778 bekannt.

Erfindungsgemäß werden sowohl die Signale aus der Kontrollzone als auch aus der Nachweiszone erfasst und miteinander durch geeignete mathematische Verfahren verrechnet. Dies geschieht beispielsweise in einer zentralen Rechnereinheit des Messgeräts. Wichtig ist erfindungsgemäß, dass beide Signale zumindest in bestimmten Konzentrationsbereichen des Analyten von der Analytkonzentration abhängig sind. Durch geeignete, im Zusammenhang mit den nachfolgenden Beispielen exemplarisch näher erläuterte mathematische Verfahren ist es so möglich, den dynamischen Messbereich der Testelemente (verglichen mit der alleinigen Auswertung des Signals in der Nachweiszone) zu erweitern.

Die Bestimmung der Analytkonzentration aus den gemessenen Signalen der Nachweis- und Kontrollzonen erfolgt wie üblich durch Vergleich der Messwerte mit entsprechenden Eichkurven, die durch Vermessen von Standardlösungen mit bekannten Analytmengen erhalten wurden. Bevorzugt gehen zur Erstellung der Eichkurve sowohl die Signale des Nachweisstrichs als auch die des Kontrollstrichs ein.

Eine mögliche Realisierung des erfindungsgemäßen Verfahrens sieht vor, dass die Reagenzien, die in der Analytnachweiszone und in der Kontrollzone zu einem detektierbaren Signal führen, unterschiedliche Affinität zum bzw. Reaktivität mit dem Analyten aufweisen. Vorzugsweise sind in der Kontrollzone niedrig affinere bzw. weniger reaktive Antikörper gegen den Analyten an der Signalbildung beteiligt als in der Analytnachweiszone.

Eine alternative mögliche Realisierung des erfindungsgemäßen Verfahrens sieht vor, dass die Reagenzien, die in der Analytnachweiszone und in der Kontrollzone zu einem detektierbaren Signal führen, aufgrund unterschiedlicher Prinzipien der Wechselwirkung mit dem Analyten oder mit anderen am Analytnachweis beteiligten Reagenzien unterschiedlich starke detektierbare Signale erzeugen. Insbesondere können in der Analytnachweiszone Antikörper gerichtet gegen den Analyten gebunden sein und in der Kontrollzone Analyt oder Analytanalogon. Es ist auch möglich, dass der in der Kontrollzone immobilisierte Bindepartner an ein anderes Epitop des Antikörpers oder an ein heterologes Strukturelement, das dem Antikörper synthetisch zugefügt wurde, bindet oder dass ein speziesspezifischer Antikörper gegen den zu immobilisierenden Antikörper in der Kontrollzone immobilisiert ist. All diese Varianten sind dem Fachmann aus dem Stand der Technik bekannt.

Eine Möglichkeit, den Messbereich bis zu sehr hohen Konzentrationen auszudehnen, ist bei Vorliegen eines so genannten "high dose hook effects" gegeben. In diesem Fall nimmt die Signalstrichintensität bei sehr hohen Analytkonzentrationen wieder ab, da die angebotene Antikörpermenge nicht mehr ausreicht, in jedem Fall einen Sandwich-Komplex zu bilden. Es bilden sich vermehrt Komplexe aus Analyt und jeweils einem Antikörper(-Konjugat). In diesem Konzentrationsbereich kann die Intensität bzw. die Konzentrationsabhängigkeit des Kontrollstrichs zu schwach sein um auswertbar zu sein. Dagegen kann die Intensitätsabnahme des Signalstrichs in Abhängigkeit von der Konzentration ausgewertet werden. Es gibt für diesen Fall also drei Auswertebereiche:

1. Intensitätszunahme des Signalstrichs in Abhängigkeit von der Konzentration

2. Intensitätsabnahme des Kontrollstrichs in Abhängigkeit von der Konzentration

3. Intensitätsabnahme des Signalstrichs in Abhängigkeit von der Konzentration

Eine automatische Unterscheidung zwischen den drei genannten Bereichen lässt sich beispielsweise durch folgenden Algorithmus erreichen:

A) Falls die Remission am Signalstrich größer ist als X1 % und die Remission am Kontrollstrich kleiner ist als Y1 %, werte nur den Signalstrich aus und nehme die Eichkurve für Analytkonzentrationen von A1 bis A2 mg/ml.

B) Falls die Remission am Signalstrich kleiner ist als X2 % und die Remission am Kontrollstrich größer ist als Y2 % und kleiner ist als Y3 %, werte nur den Kontrollstrich aus und nehme die Eichkurve für Analytkonzentrationen von A3 bis A4 mg/ml.

C) Falls die Remission am Signalstrich größer ist als X3 % und die Remission am Kontrollstrich größer ist als Y4 %, werte nur den Signalstrich aus und nehme die Eichkurve für Analytkonzentrationen von A5 bis A6 mg/ml.

Die Erfindung wird anhand der nachfolgenden Beispiele und Figuren näher erläutert. Obwohl in den Beispielen exemplarisch nur immunologische Testdevices, die mit goldmarkierten Bindepartnern arbeiten und remissionsfotometrisch vermessen werden, gezeigt sind, ist die Erfindung nicht hierauf beschränkt. Neben immunologischen Wechselwirkungen, die ja auf Antigen- (bzw. Hapten-) Antikörper Bindepaaren beruhen, sind auch andere Bindepaare möglich, insbesondere HormonRezeptor, Zucker-Lectin, Nukleinsäure-komplementäre Nukleinsäure, Biotin-(Strept-)Avidin und dergleichen mehr. Außer Goldmarkierungen sind auch andere partikuläre Markierungen möglich, wie z. B. die Verwendung von farbigen Latices, Polymerlabel, oder Halbleiter-Nanokristallen (so genannten Quantum Dots) oder anderer Metall(sol)label, sowie nicht-partikuläre Markierungen (Enzym-, Radioisotop-, Fluoreszenzlabel) und dergleichen mehr. Je nach verwendetem Label sind naturgemäß auch andere Detektionsmethoden erforderlich und möglich (z. B. Fluoreszenzmessung, Radioaktivitätsmessung, Enzymaktivitätsbestimmung etc.). Dem Fachmann sind diese Variationen in einer Vielzahl von Ausführungsmöglichkeiten bekannt.

In Figur 1 ist schematisch eine bevorzugte Ausführungsform eines erfindungsgemäß einsetzbaren Testdevices in Form eines immunochromatographischen Teststreifens abgebildet.

In Figur 2 sind die relativen Remissionswerte (R in %) bei der Auswertung des Nachweisstriches (NS) und des Kontrollstriches (KS) in Abhängigkeit von der Troponin T-Konzentration in der Probe (C in ng/ml) abgebildet.

In Figur 3 sind die Kalibrationskurven für einen Troponin T-Teststreifen wiedergegeben, die sich durch Anwendung der in Beispiel 2 näher erläuterten Algorithmen a) (Stand der Technik) und b) (Erfindung) auf die Messwerte aus Figur 2 ergeben.

In Figur 4 ist die Kalibrationskurve für einen Troponin T-Teststreifen wiedergegeben, die sich durch Anwendung des in Beispiel 2 näher erläuterten Algorithmus c) (Erfindung) auf die Messwerte aus Figur 2 ergibt.

In Figur 5 sind die relativen Remissionswerte (R in %) bei der Auswertung des Nachweisstriches (NS) und des Kontrollstriches (KS) in Abhängigkeit von der NT-proBNP-Konzentration in der Probe (C in ng/ml) abgebildet.

In Figur 6 sind die Kalibrationskurven für einen NT-proBNP-Teststreifen wiedergegeben, die sich durch Anwendung der in Beispiel 3 näher erläuterten Algorithmen a) (Stand der Technik) und b) (Erfindung) auf die Messwerte aus Figur 5 ergeben.

In Figur 7 sind die relativen Remissionswerte (R in %) bei der Auswertung des Nachweisstriches (NS) und des Kontrollstriches (KS) in Abhängigkeit von der D-Dimer-Konzentration in der Probe (c in $\mu$g/ml) in drei Teilfiguren (die jeweils unterschiedliche Konzentrationsbereiche abdecken) abgebildet.

Die Ziffern in den Figuren bedeuten:

1 Probenaufgabezone
2 Erythrozytenabtrennzone
3 Detektionszone
4 Saugzone
5 Trägermaterial
6 Probenaufgabematrix ("Biotinvlies" und "Goldvlies")
7 Erythrozytenabtrennmatrix
8 Detektionsmatrix
9 1. strichförmige Immobilisierungszone (Nachweisstrich; Analytnachweiszone)
10 2. strichförmige Immobilisierungszone (Kontrollstrich; Kontrollzone)
11 Saugmatrix

**Beispiele**

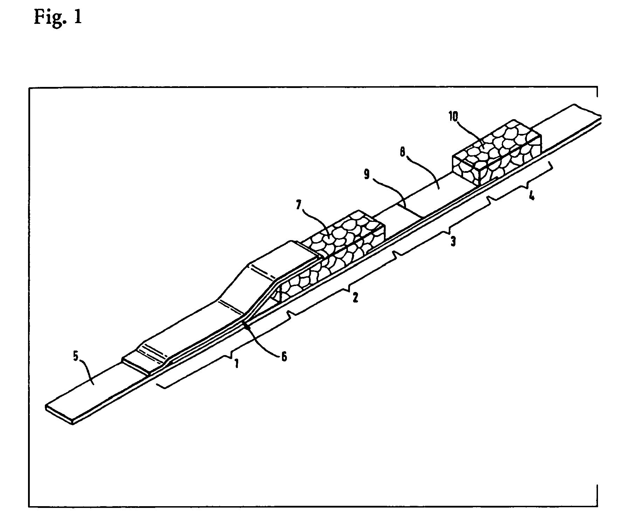

**1) Herstellung eines Testdevices zur Bestimmung von Antigenen aus Vollblut (vgl. Fig. 1)**

Das Testdevice (Fig. 1) besteht aus einem Trägermaterial (5) auf das eine Probenaufgabezone (1), eine Erythrozytenabtrennzone (2), eine Detektionszone (3) und eine Saugzone (4) aufgebracht sind. In der Probenaufgabezone (1) ist eine Probenaufgabematrix (6) angeordnet, die eine Erythrozytenabtrennmatrix (7) teilweise überlappt. Die Erythrozytenabtrennmatrix (7) ihrerseits überlappt etwas die Detektionsmatrix (8) (Detektionszone), auf der Polystreptavidin in Form eines Striches (9) als Nachweisstrich und Antigen bzw. eine Antigenanalogon, z. B. als synthetisches bzw. rekombinantes Antigen-Peptid, in Form eines Striches als Kontrollstrich immobilisiert sind. Eine Saugmatrix (10) überlappt etwas die Detektionsmatrix (8). In der Probenaufgabematrix (6) sind alle diejenigen Reagenzien untergebracht, die zur Bildung eines Komplexes mit dem nachzuweisenden Analyten erforderlich sind. Im vorliegenden Fall besteht die Probenaufgabezone aus 2 übereinander liegenden Vliesen, wobei das erste ("Goldvlies") mit einem goldmarkierten Antikörper gegen den Analyten getränkt ist und das zweite Vlies ("Biotinvlies") einen biotinylierten Antikörper gegen den Analyten enthält. Analyt ist hier ein in Blut vorkommendes Antigen, insbesondere Troponin T, NT-proBNP oder D-Dimer.

Als Trägerschicht 5 wird eine 350 $\mu$m dicke Polyesterfolie (Pütz) verwendet. Als "Goldvlies" bzw. "Biotinvlies" der Probenaufgabematrix 6 wird ein 360 μm dickes Polyestervlies (Roche Diagnostics) verwendet. Als Erythrozytenabtrennmatrix 7 wird ein 1.8 mm dickes Glasfaservlies (Roche Diagnostics) verwendet. Als Detektionsmatrix 8 dient eine 140 μm dicke Nitrocellulosemembran (Sartorius). Als Saugmatrix 10 dient ein 1.8 mm dickes Glasfaservlies (Roche Diagnostics). Die einzelnen Komponenten (6, 7, 8, 11) werden wie in Fig. 1 gezeigt mittels Schmelzkleber leicht überlappend auf die Trägerschicht 5 aufgeklebt.

Die Tränkrezepturen für die "Gold- und Biotinvliese" der aufgeführten Beispiele sind:

proBNP-Teststreifen:

"Biotinvlies":   100 mM Hepes pH 7.4, 0.1 % Tween®, biotinylierter Antikörper gegen den Analyten

"Goldvlies":   100 mM Hepes pH 7.4, Antikörper gegen den Analyten als Goldkonjugat

Troponin T-Teststreifen:

"Biotinvlies":   100 mM MES pH 5.6 biotinylierter Antikörper gegen den Analyten

"Goldvlies":   100 mM Bernsteinsäure pH 5.6, 0.1 % Tween Antikörper gegen den Analyten als Goldkonjugat

D-Dimer-Teststreifen:

"Biotinvlies":   100 mM Hepes pH 7.4, 0.1 % Tween®, biotinylierter Antikörper gegen den Analyten
"Goldvlies":   100 mM Hepes pH 7.4, Antikörper gegen den Analyten als Goldkonjugat

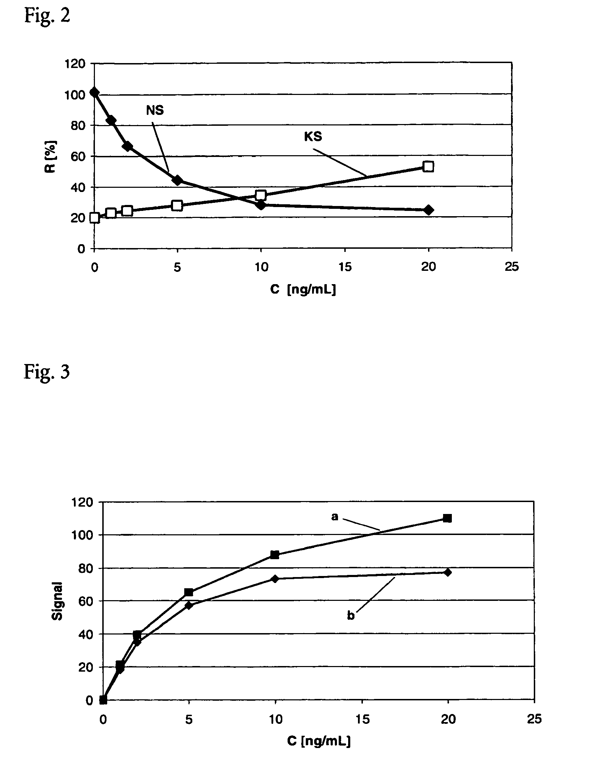

## 2) Auswertung eines Teststreifens für die Troponin T-Bestimmung (Fig. 2 bis 4)

Troponin T-Teststreifen gemäß Beispiel 1 wurden mit Vollblutproben, denen rekombinant hergestelltes Troponin T in unterschiedlichen Mengen zugesetzt war, beschickt. Die Streifen wurden sowohl nach zwei erfindungsgemäßen Verfahren (Varianten b) und c), siehe unten) als auch zu Vergleichszwecken nach dem herkömmlichen Verfahren (Variante a)) ausgewertet. Die Remission wurde dabei für den Nachweisstrich (NS) (9) und den Kontrollstrich (KS) mit einem herkömmlichen, auf einer CCD-Kamera aufbauenden Messgerät (Cardiac Reader, Roche Diagnostics GmbH) bestimmt und die Signale nach folgendem Algorithmus verrechnet:

a)

$$\left| \text{Rem NS} \, (0) - \text{Rem NS} \, (c) \right|$$

b)

$$\left| \text{Rem KS} \, (0) - \text{Rem KS} \, (c) \right| + \left| \text{Rem NS} \, (0) - \text{Rem NS} \, (c) \right|$$

c)

$$\left| \text{Rem KS} \, (0) - \text{Rem NS} \, (c) \right| * \left| \text{Rem NS} \, (0) - \text{Rem NS} \, (c) \right|$$

wobei die Algorithmusalternative a) nur die übliche Auswertung des Nachweisstriches gemäß Stand der Technik darstellt. Erfindungsgemäß werden bei b) und c) zur Auswertung sowohl das Nachweisstrich- als auch das Kontrollstrichsignal herangezogen.

In den Formeln bedeuten:

Rem NS (0) Remission des Nachweisstriches in % bei Analytkonzentration 0

Rem NS (c) Remission des Nachweisstriches in % bei Analytkonzentration c
Rem KS (0) Remission des Kontrollstriches in % bei Analytkonzentration 0
Rem KS (c) Remission des Kontrollstriches in % bei Analytkonzentration c

In Figur 2 sind die relativen Remissionswerte (R in %) bei der Auswertung des Nachweisstriches (NS) und des Kontrollstriches (KS) in Abhängigkeit von der Troponin T-Konzentration in der Probe (c in ng/ml) abgebildet. Figur 2 verdeutlicht die Signalabnahme (Remissionszunahme) des Kontrollstriches mit zunehmender Analytkonzentration bei gleichzeitiger Signalzunahme (Remissionsabnahme) des Nachweisstriches.

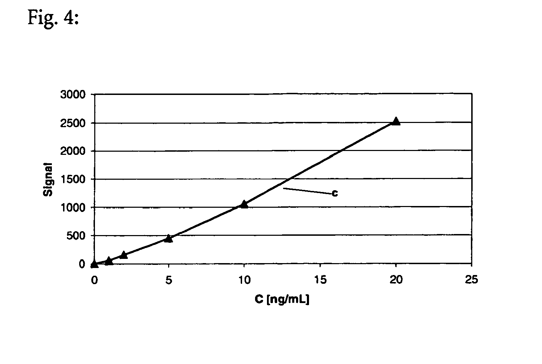

In Figur 3 sind die Kalibrationskurven für einen Troponin T-Teststreifen wiedergegeben, die sich durch Anwendung der oben erläuterten Algorithmen a) (Stand der Technik) und b) (Erfindung) auf die Messwerte aus Figur 2 ergeben. In Figur 4 ist die Kalibrationskurve für einen Troponin T-Teststreifen wiedergegeben, die sich durch Anwendung des oben näher erläuterten Algorithmus c) (Erfindung) auf die Messwerte aus Figur 2 ergibt. Während mit der alleinigen Auswertung des Nachweisstriches (Algorithmus a)) bei diesem Test Konzentrationsbestimmungen nur bis ca. 10 ng/ml durchführbar sind, sind durch Auswertung des KS und NS nach den Algorithmen b) und c) noch Konzentrationen von mehr als 20 ng/ml bestimmbar.

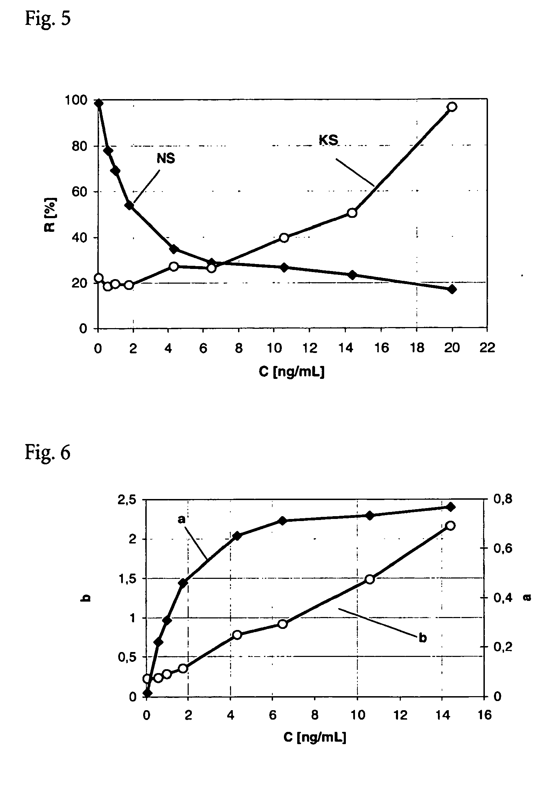

**3) Auswertung eines Teststreifens für die NT-proBNP-Bestimmung (Fig. 5 bis 6)**

NT-proBNP-Teststreifen gemäß Beispiel 1 wurden mit Vollblutproben, denen synthetisches NT-proBNP in unterschiedlichen Mengen zugesetzt war, beschickt. Die Streifen wurden sowohl nach dem erfindungsgemäßen Verfahren (Varianten b), siehe unten) als auch zu Vergleichszwecken nach dem herkömmlichen Verfahren (Variante a)) ausgewertet. Die Remission wurde dabei für den Nachweisstrich (NS) (9) und den Kontrollstrich (KS) mit einem herkömmlichen, auf einer CCD-Kamera aufbauenden Messgerät (Cardiac Reader, Roche Diagnostics GmbH) bestimmt und die Signale nach folgendem Algorithmus verrechnet:

a)

$$1 - \text{Rem NS (c)}$$

b)

$$\text{Rem KS (c)} : \text{Rem NS (c)}$$

wobei die Algorithmusalternative a) nur die übliche Auswertung des Nachweisstriches gemäß Stand der Technik darstellt. Erfindungsgemäß wird bei b) zur Auswertung sowohl das Nachweisstrich- als auch das Kontrollstrichsignal herangezogen.

Die Abkürzungen in den Formeln haben dieselbe Bedeutung wie in Beispiel 2.

In Figur 5 sind die relativen Remissionswerte (R in %) bei der Auswertung des Nachweisstriches (NS) und des Kontrollstriches (KS) in Abhängigkeit von der NT-proBNP-Konzentration in der Probe (c in ng/ml) abgebildet. Figur 5 verdeutlicht die Signalabnahme (Remissionszunahme) des Kontrollstriches mit zunehmender Analytkonzentration bei gleichzeitiger Signalzunahme (Remissionsabnahme) des Nachweisstriches.

In Figur 6 sind die Kalibrationskurven für einen NT-proBNP-Teststreifen wiedergegeben, die sich durch Anwendung der oben erläuterten Algorithmen a) (Stand der Technik) und b) (Erfindung) auf die Messwerte aus Figur 5 ergeben. Während mit der alleinigen Auswertung des Nachweisstriches (Algorithmus a)) bei diesem Test Konzentrationsbestimmungen nur bis ca. 6 ng/ml durchführbar sind, sind durch Auswertung des KS und NS nach dem Algorithmus b) noch Konzentrationen von mehr als 14 ng/ml bestimmbar.

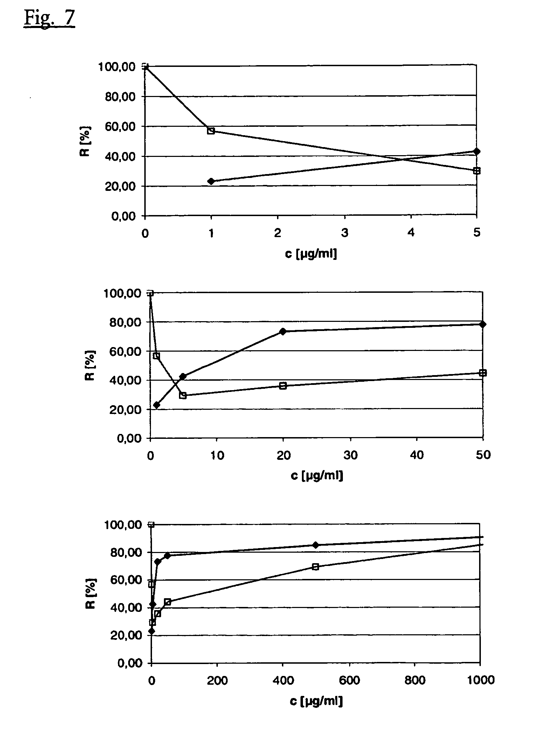

**4) Auswertung eines Teststreifens für die D-Dimer-Bestimmung (Fig. 7)**

D-Dimer-Teststreifen gemäß Beispiel 1 (am Nachweisstrich wird biotinylierter Antikörper, der gegen den Analyten gerichtet ist, immobilisiert; der Kontrollstrich dagegen besteht aus immobilisierten Fibrinfragmenten, die das D-Dimer-Strukturelement enthalten; an diesen kann freier Gold-Konjugat-Antikörper gebunden werden) wurden mit Vollblutproben, denen D-Dimerhaltige Fibrinfragmente in unterschiedlichen Mengen zugesetzt war, beschickt. Die Streifen wurden sowohl nach dem erfindungsgemäßen Verfahren (Variante b, siehe unten) als auch zu Vergleichszwecken nach dem herkömmlichen Verfahren (Variante a) ausgewertet. Die Remission wurde dabei für den Nachweisstrich (NS) (9) und den Kontrollstrich (KS) mit einem herkömmlichen, auf einer CCD-Kamera aufbauenden Messgerät (Cardiac Reader, Roche Diagnostics GmbH) bestimmt und die Signale nach folgendem Algorithmus verrechnet:

a) Rem NS (c)

b)

1) Falls die Remission am Signalstrich größer ist als 30 % und die Remission am Kontrollstrich kleiner ist als 40 %, werte nur den Signalstrich aus (Rem NS (c)) und nehme die Eichkurve für Analytkonzentrationen von 0 bis 3 $\mu$g/ml.

2) Falls die Remission am Signalstrich kleiner ist als 50 % und die Remission am Kontrollstrich größer ist als 40 % und kleiner ist als 70 %, werte nur den Kontrollstrich aus (Rem KS (c)) und nehme die Eichkurve für Analytkonzentrationen von 3 bis 20 $\mu$g/ml.

3) Falls die Remission am Signalstrich größer ist als 30 % und die Remission am Kontrollstrich größer ist als 70 %, werte nur den Signalstrich aus (Rem NS (c)) und nehme die Eichkurve für Analytkonzentrationen von 20 $\mu$g/ml bis 1000 $\mu$g/ml.

wobei die Algorithmusalternative a) nur die übliche Auswertung des Nachweisstriches gemäß Stand der Technik darstellt. Erfindungsgemäß wird bei b) zur Auswertung sowohl das Nachweisstrich- als auch das Kontrollstrichsignal herangezogen.

Die Abkürzungen in den Formeln haben dieselbe Bedeutung wie in Beispiel 2.

In Figur 7 sind die relativen Remissionswerte (R in %) bei der Auswertung des Nachweisstriches (NS) und des Kontrollstriches in Abhängigkeit von der D-Dimer-Konzentration in der Probe (c in $\mu$g/ml) abgebildet.

Bis zu einer D-Dimer-Konzentration von ca. 3 $\mu$g/ml kann die Auswertung wie üblich über die Remissions-Abnahme (Intensitätszunahme) des Signalstrichs erfolgen. Bis ca. 20 $\mu$g/ml kann die Remissions-Zunahme (Intensitätsabnahme) des Kontrollstriches ausgewertet werden. Bei einer Analytkonzentration von über 20 $\mu$g/ml ist die Konzentrationsabhängigkeit des Kontrollstrichsignals zu gering. Ab ca. 20 $\mu$g/ml bis > 1000 $\mu$g/ml kann die Remissions-Zunahme (Intensitätsabnahme) des Signalstrichs für die Auswertung verwendet werden. Da die Messbereichsuntergrenze < 0.5 $\mu$g/ml ist, ist somit ein Dynamik-Faktor von > 1000 erreichbar.

## Patentansprüche

1. Verfahren zur Erweiterung des dynamischen Messbereiches zu höheren Analytkonzentrationen, bei denen Sättigungseffekte den Messbereich limitieren, insbesondere der "High-Dose-Hook-Effect" auftritt, ohne Beeinflussung der unteren Nachweisgrenze eines auf spezifischen Bindereaktionen basierenden, insbesondere immunologischen, Testelementes, welches sowohl eine Analytnachweiszone als auch eine Kontrollzone aufweist, wobei:

a) eine Probe mit dem Testelement und mit für einen Analyten spezifischen Reagenzien in Kontakt gebracht wird;
b) der Analyt - sofern in der Probe vorhanden - durch Wechselwirkung mit spezifischen Reagenzien zu einem in der Analytnachweiszone detektierbaren Signal führt, welches von der Menge an Analyt in der Probe abhängig ist;
c) ein Teil der spezifischen Reagenzien, der nicht mit dem Analyten oder den Reagenzien in der Analytnachweiszone in Wechselwirkung getreten ist, in der Kontrollzone zu einem detektierbaren Signal führt, welches ebenfalls von der Menge an Analyt in der Probe abhängig ist
d) die Signale in der Analytnachweiszone und in der Kontrollzone gemessen werden;
e) beide Signale durch einen geeigneten Algorithmus verrechnet werden;
f) das Ergebnis hieraus mit einer Eichkurve verglichen wird; und
g) die Analytkonzentration bestimmt wird.

2. Verfahren gemäß Anspruch 1, **dadurch gekennzeichnet, dass** in Schritt e) die beiden Signale miteinander verrechnet werden.

3. Verfahren gemäß Anspruch 1, **dadurch gekennzeichnet, dass** mindestens zwei verschiedene Eichkurven für die Analytnachweiszone und die Kontrollzone vorliegen, und in Abhängigkeit von den in Schritt d) gemessenen Signalen entweder nur das Signal der Analytnachweiszone oder nur das Signal der Kontrollzone mit der entsprechenden Eichkurve verglichen wird.

**4.** Verfahren gemäß Anspruch 1 oder 2, **dadurch gekennzeichnet, dass** zur Erstellung der Eichkurve sowohl die Signale des Nachweisstrichs als auch die des Kontrollstrichs eingehen.

**5.** Verfahren gemäß einem der Ansprüche 1 bis 4, **dadurch gekennzeichnet, dass** die Reagenzien, die in der Analytnachweiszone und in der Kontrollzone zu einem detektierbaren Signal führen, unterschiedliche Affinität bzw. Reaktivität zum Analyten aufweisen.

**6.** Verfahren gemäß Anspruch 5, **dadurch gekennzeichnet, dass** in der Kontrollzone niedrig affinere bzw. weniger reaktive Antikörper gegen den Analyten an der Signalbildung beteiligt sind als in der Analytnachweiszone.

**7.** Verfahren gemäß einem der Ansprüche 1 bis 4, **dadurch gekennzeichnet, dass** die Reagenzien, die in der Analytnachweiszone und in der Kontrollzone zu einem detektierbaren Signal führen, aufgrund unterschiedlicher Prinzipien der Wechselwirkung mit dem Analyten oder mit anderen am Analytnachweis beteiligten Reagenzien unterschiedlich starke detektierbare Signale erzeugen.

**8.** Verfahren gemäß Anspruch 7, **dadurch gekennzeichnet, dass** in der Analytnachweiszone Antikörper gerichtet gegen den Analyten gebunden sind und in der Kontrollzone Analyt oder Analytanalogon gebunden ist.

**9.** Verwendung eines auf spezifischen Bindereaktionen basierenden, insbesondere immunologischen, Testelementes zur Erweiterung des dynamischen Messbereiches zu höheren Analytkonzentrationen, bei denen Sättigungseffekte den Messbereich limitieren, insbesondere der "High-Dose-Hook-Effect" auftritt, ohne Beeinflussung der unteren Nachweisgrenze, wobei das Testelement sowohl eine Analytnachweiszone als auch eine Kontrollzone aufweist und das in der Analytnachweiszone detektierte Signal und das in der Kontrollzone detektierte Signal jeweils abhängig von der Menge des Analyten in der Probe ist und beide Signale durch einen geeigneten Algorithmus verrechnet werden.

**10.** Verwendung nach Anspruch 9, wobei:

a) eine Probe mit dem Testelement und mit für einen Analyten spezifischen Reagenzien in Kontakt gebracht wird;
b) die Signale in der Analytnachweiszone und in der Kontrollzone gemessen werden;
c) beide Signale miteinander verrechnet werden;
d) das Ergebnis hieraus mit einer Eichkurve verglichen wird; und
e) die Analytkonzentration bestimmt wird.

**Claims**

**1.** Method for extending the dynamic measuring range towards higher analyte concentrations, in which saturation effects limit the measuring range and in particular the high dose hook effect occurs, without affecting the lower detection limit of a test element based on specific binding reactions and in particular an immunological test element which has an analyte detection zone as well as a control zone, wherein:

a) a sample is brought into contact with the test element and with specific reagents for the analyte;
b) the analyte, if present in the sample, results in a detectable signal in the analyte detection zone due to interaction with specific reagents, said signal being dependent on the amount of analyte in the sample;
c) a portion of the specific reagents which has not interacted with the analyte or with the reagents in the analyte detection zone leads to a detectable signal in the control zone which is also dependent on the amount of analyte in the sample;
d) the signals in the analyte detection zone and in the control zone are measured;
e) both signals are offset using a suitable algorithm;
f) the result thereof is compared with a calibration curve; and
g) the analyte concentration is determined.

**2.** Method according to claim 1, **characterized in that** the two signals are offset against one another in step e).

3. Method according to claim 1, **characterized in that** at least two different calibration curves are present for the analyte detection zone and the control zone and, depending on the signals measured in step d), either only the signal of the analyte detection zone or only the signal of the control zone is compared with the corresponding calibration curve.

4. Method according to claim 1 or 2, **characterized in that** the signals of the detection line as well as those of the control line are used to establish the calibration curve.

5. Method according to one of the claims 1 to 4, **characterized in that** the reagents which lead to a detectable signal in the analyte detection zone and in the control zone have a different affinity for or reactivity to the analyte.

6. Method according to claim 5, **characterized in that** the antibodies against the analyte that are involved in the signal formation in the control zone have a lower affinity or are less reactive than those in the analyte detection zone.

7. Method according to one of the claims 1 to 4, **characterized in that** the reagents which lead to a detectable signal in the analyte detection zone and in the control zone generate detectable signals of different strengths due to different principles of interaction with the analyte or with other reagents involved in the analyte detection.

8. Method according to claim 7, **characterized in that** antibodies directed against the analyte are bound in the analyte detection zone and the analyte or an analyte analogue is bound in the control zone.

9. Use of a test element and in particular immunological test element based on specific binding reactions to extend the dynamic measuring range towards higher analyte concentrations, in which saturation effects limit the measuring range and in particular the high dose hook effect occurs, without affecting the lower detection limit, wherein the test element has an analyte detection zone as well as a control zone and the signal detected in the analyte detection zone and the signal detected in the control zone each depend on the amount of analyte in the sample and both signals are offset by a suitable algorithm.

10. Use according to claim 9, wherein:

   a) a sample is brought into contact with the test element and with specific reagents for the analyte;
   b) the signals in the analyte detection zone and in the control zone are measured;
   c) both signals are offset against one another;
   d) the result thereof is compared with a calibration curve; and
   e) the analyte concentration is determined.

**Revendications**

1. Procédé pour élargir la plage de mesure dynamique dans le sens de concentrations d'analytes supérieures dans lesquelles des effets de saturation limitent la plage de mesure, en particulier dans lesquelles se manifeste le « High-Dose-Hook-Effect », sans influencer la limite de décèlement inférieure d'un élément d'essai en particulier immunologique se basant sur des réactions de liaison spécifiques, qui présente aussi bien une zone de décèlement d'analyte qu'une zone témoin, dans lequel :

   a) on amène un échantillon en contact avec l'élément d'essai et avec des réactifs spécifiques pour un analyte ;
   b) l'analyte - pour autant qu'il soit présent dans l'échantillon - via une interaction avec des réactifs spécifiques donne lieu à un signal détectable dans la zone de décèlement de l'analyte, qui dépend de la quantité de l'analyte dans l'échantillon ;
   c) une partie des réactifs spécifiques, qui n'est pas entrée en interaction avec l'analyte ou avec les réactifs dans la zone de décèlement de l'analyte, donne lieu dans la zone témoin à un signal détectable qui dépend également de la quantité de l'analyte dans l'échantillon ;
   d) on mesure les signaux dans la zone de décèlement de l'analyte et dans la zone témoin ;
   e) on traite les deux signaux via un algorithme approprié ;
   f) on compare à une courbe d'étalonnage les résultats que l'on obtient ; et
   g) on détermine la concentration de l'analyte.

2. Procédé selon la revendication 1, **caractérisé en ce que,** à l'étape e), les deux signaux sont traités conjointement.

**3.** Procédé selon la revendication 1, **caractérisé en ce qu'**au moins deux courbes d'étalonnage différentes sont présentes pour la zone de décèlement de l'analyte et pour la zone témoin, et, en fonction des signaux mesurés à l'étape d), on compare soit seulement le signal de la zone de décèlement de l'analyte, soit seulement le signal de la zone témoin à la courbe d'étalonnage correspondante.

**4.** Procédé selon la revendication 1 ou 2, **caractérisé en ce que**, pour la construction de la courbe d'étalonnage, on inclut aussi bien les signaux du frottis de décèlement que ceux du frottis témoin.

**5.** Procédé selon l'une quelconque des revendications 1 à 4, **caractérisé en ce que** les réactifs, qui donnent lieu à un signal détectable dans la zone de décèlement de l'analyte et dans la zone témoin, présentent une affinité respectivement une réactivité différente vis-à-vis de l'analyte.

**6.** Procédé selon la revendication 5, **caractérisé en ce que** les anticorps vis-à-vis de l'analyte, qui contribuent à la formation du signal dans la zone témoin, manifestent une affinité respectivement une réactivité inférieure à ceux dans la zone de décèlement de l'analyte.

**7.** Procédé selon l'une quelconque des revendications 1 à 4, **caractérisé en ce que** les réactifs, qui donnent lieu à un signal détectable dans la zone de décèlement de l'analyte et dans la zone témoin, génèrent, sur base de principes différents de l'interaction avec l'analyte ou avec d'autres réactifs contribuant au décèlement de l'analyte, des signaux détectables dont l'intensité diffère.

**8.** Procédé selon la revendication 7, **caractérisé en ce que**, dans la zone de décèlement de l'analyte, sont liés des anticorps dirigés contre l'analyte et dans la zone témoin, est lié l'analyte ou un analogue de l'analyte.

**9.** Utilisation d'un élément d'essai, en particulier immunologique, se basant sur des réactions de liaison spécifiques, pour élargir la plage de mesure dynamique dans le sens de concentrations d'analytes supérieures dans lesquelles des effets de saturation limitent la plage de mesure, en particulier dans lesquelles se manifeste le « High-Dose-Hook-Effect », sans influencer la limite de décèlement inférieure, dans laquelle l'élément d'essai présente aussi bien une zone de décèlement de l'analyte qu'une zone témoin, le signal détecté dans la zone de décèlement de l'analyte et le signal détecté dans la zone témoin dépendant respectivement de la quantité de l'analyte dans l'échantillon et les deux signaux étant traités via un algorithme approprié.

**10.** Utilisation selon la revendication 9, dans laquelle :

a) on amène un échantillon en contact avec l'élément d'essai et avec des réactifs spécifiques pour un analyte ;
b) on mesure les signaux dans la zone de décèlement de l'analyte et dans la zone témoin ;
c) on soumet les deux signaux à une compensation réciproque ;
d) on compare à une courbe d'étalonnage le résultat que l'on obtient ; et
e) on détermine la concentration de l'analyte.

Fig. 1

Fig. 2

Fig. 3

Fig. 4:

Fig. 5

Fig. 6

Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9706439 A
- EP 0291194 A
- US 5591645 A
- US 4861711 A
- US 5141850 A
- US 6506612 B
- US 5458852 A
- US 5073484 A
- US 6248597 B
- US 5585241 A
- US 4743542 A
- US 4595661 A
- WO 0031538 A
- US 20030119204 A
- US 5717778 A

### In der Beschreibung aufgeführte Nicht-Patentliteratur

- **J. HAMPL et al.** Upconverting Phosphor Reporters in Immunochromatographic Assays. *Analytical Biochemistry,* 2001, vol. 288, 176-187